# EUROPÄISCHE PATENTSCHRIFT

(11) **EP 1 893 272 B1**
(45) Veröffentlichungstag und Bekanntmachung des Hinweises auf die Patenterteilung: **25.01.2017**
(21) Anmeldenummer: 06725435.9
(22) Anmeldetag: 30.03.2006
(51) Int. Cl.: A61M 16/04, A61B 17/34, A61M 25/06

(54) **EINFÜHRHILFE FÜR DIE PERKUTANE TRACHEOSTOMIE**
INSERTION AID FOR PERCUTANEOUS TRACHEOSTOMY
AUXILIAIRE D'INTRODUCTION DESTINÉ À UNE TRACHÉOSTOMIE PERCUTANÉE

(30) Priorität: 10.05.2005 DE 102005021470
(43) Veröffentlichungstag der Anmeldung: 05.03.2008
(73) Patentinhaber: TRACOE medical GmbH, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2006/061184
(87) Internationale Veröffentlichungsnummer: WO 2006/120077

(56) Entgegenhaltungen:
- EP-A- 1 099 451
- US-A- 4 502 482
- US-A- 4 637 388
- US-A- 5 058 580
- US-A- 5 824 002

## Beschreibung

Die vorliegende Erfindung betrifft eine Vorrichtung zum Einbringen einer Trachealkanüle in ein Tracheostoma. Aus der US-4,637,388 A1 ist ein Obturator als Teil eines Trachealtubus-Aufbaus bekannt, der der Einbringung eines Endotrachealtubus in die Trachea eines Patienten über Mund und Rachenraum erleichtern soll.

Im Einzelnen beschreibt die US 4,637,388 einen Obturator als Teil eines Trachealtubus-Sets, der leicht durch die Stimmbandritze hindurchgeleitet werden kann, wobei der Obturator eine weiche, flexible, abgerundete, konische Spitze aufweist, die das Tubusende während des Einführens bedeckt. Der Obturator dient als Einführhilfe der Einbringung einer Trachealkanüle durch den Mund- bzw. Rachenraum. Die konische Spitze hat in einem ersten Zustand einen kleinen Basisdurchmesser und in einem zweiten Zustand einen größeren Basisdurchmesser. Ein an die konische Spitze angrenzender Abschnitt hat einen Durchmesser der dem Innendurchmesser einer mit dem Obturator einzuführenden Trachealkanüle entspricht, wobei der kleine Basisdurchmesser der konischen Spitze höchstens gleich dem Innendurchmesser der Trachealkanüle ist. Der große Basisdurchmesser der konischen Spitze ist hingegen größer als der Innendurchmesser der Trachealkanüle.

Die Tracheotomie gehört zu den ältesten operativen Techniken in der Geschichte der Medizin. Die Ursprünge dieser Technik gehen bis in die Antike zurück. Bei der klassischen Tracheotomie wird chirurgisch unterhalb des Kehlkopfes, typischerweise zwischen 1. und 2. bzw. 2. und 3. Trachealring, ein künstlicher Zugang zur Luftröhre geschaffen. Um diese künstliche Öffnung zwecks Beatmung aufrechtzuerhalten, wird eine sogenannte Trachealkanüle eingesetzt. Medizinische Indikationen, wie z. B. der Notwendigkeit einer Langzeitbeatmung, machen diese Technik auch heute noch notwendig. Alternativ zur chirurgischen Technik wurden innerhalb der letzten Jahrzehnte auch minimalinvasive Punktionstechniken entwickelt. So finden insbesondere die perkutane Dilatationstracheotomie nach Ciaglia, die Dilatationstracheotomie nach Griggs und die translaryngeale Tracheotomie nach Fantoni breite Anwendung.

Bei der perkutanen Dilatationstracheotomie nach Ciaglia wird die Trachea an geeigneter Stelle zunächst mit einer Stahlkanüle punktiert. Damit dabei die empfindliche tracheale Rückwand nicht verletzt wird, findet dieser Vorgang typischerweise unter bronchoskopischer Überwachung statt. Die korrekte Lage der Kanülenspitze kann durch Luftaspiration in eine aufgesetzte, mit Flüssigkeit gefüllte Spritze überprüft werden. Bei korrekter Lage wird ein über der Stahlkanüle befindlicher Teflonkatheter 1 bis 2 cm distal in die Trachea eingeführt. Nach Entfernung der Stahlkanüle wird ein J-förmiger Führungsdraht (ca. 1,3 mm Durchmesser) durch diesen Teflonkatheter in die Trachea vorgeschoben. Danach kann der Teflonkatheter entfernt werden. Stattdessen wird nun ein schlauchförmiger Kunststoffkatheter mit Sicherheitsstop zwecks Armierung über den Führungsdraht geschoben. Über den armierten Führungsdraht werden nun ein oder nacheinander mehrere Dilatatoren zur Erweiterung der Punktionsöffnung mit Hilfe einer rotierenden Bewegung in das Tracheallumen geschoben. Aufgrund der konischen Form der Dilatatoren weitet sich das Gewebe auf, so daß die mit Gleitmittel benetzte Trachealkanüle mit Hilfe einer speziellen Einführhilfe (Obturator) über den Führungsdraht eingesetzt werden kann. Die korrekte Lage wird mit dem Bronchoskop überprüft. Anschließend kann der Führungsdraht entfernt werden und ein an der Trachealkanüle befindlicher Ballon zur Abdichtung der Luftröhre mit Luft gefüllt werden.

Bei der Dilatationstracheotomie nach Griggs wird anstelle der konusförmigen Dilatatoren eine Zange zur Aufweitung des Gewebes verwendet.

Bei der minimalinvasiven Dilatationstechnik ist das angelegte Tracheostoma sehr eng. Um die Trachealkanüle dennoch einführen zu können, verwendet man eine Einführhilfe, deren Spitze ähnlich wie bei den Dilatatoren konisch angespitzt ist. Die Einführhilfe ist ein flexibler Stab oder Schlauch, dessen Länge so bemessen ist, daß er vollständig durch das Lumen der Trachealkanüle hindurchgeschoben werden kann, so daß am distalen Ende die konische Spitze aus der Trachealkanüle herausragt. In diesem Zustand wird die Trachealkanüle in das Tracheostoma eingeführt und durch Engstellen hindurchgedrückt, wobei die konische Spitze die Engstelle entsprechend aufweitet.

Nach dem Einführen Trachealkanüle muß selbstverständlich die Einführhilfe wieder aus der Kanüle herausgezogen werden. Daraus ergibt sich zwangsweise, daß der Durchmesser der gesamten Einführhilfe einschließlich des Bereichs der konischen Spitze kleiner sein muß als der Innendurchmesser der Kanüle. Da die Kanüle eine gewisse Stabilität aufweisen muß, hat sie auch eine entsprechende Wandstärke von mindestens 0,5 bis 1 mm. Dies wiederum bedeutet, daß es an der Basis der konischen Spitze der Einführhilfe oder, falls diese weiter über das distale Ende der Trachealkanüle hinausragt, an dem entsprechenden Schaftabschnitt der Einführhilfe einen stufenförmigen Übergang vom Innendurchmesser der Kanüle auf den Außendurchmesser gibt. Mit anderen Worten, die distale Stirnfläche der Trachealkanüle liegt frei. Dies bedeutet wiederum, daß gerade an Engstellen des Tracheostomas die konische Spitze zwar zunächst eine Aufweitung bis auf den Innendurchmesser der Kanüle bewirkt, die verbleibende Aufweitung jedoch durch die hierfür nicht besonders ausgestaltete stirnseitige Fläche der Trachealkanüle erfolgen muß. Dies kann unter Umständen zu Verletzungen führen und erschwert auf jeden Fall das Einführen der Kanüle erheblich.

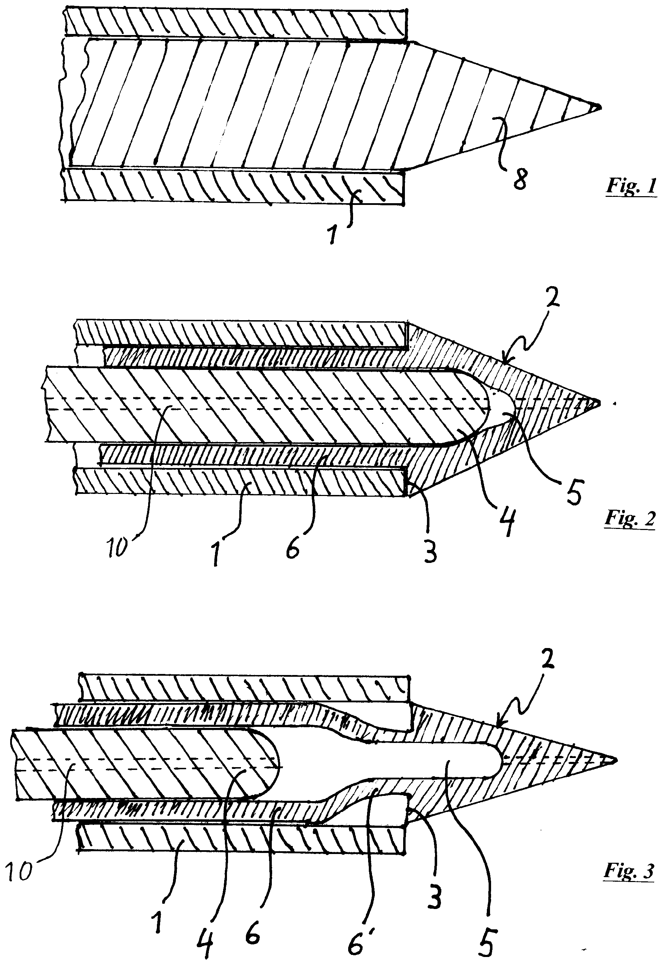

Figur 1 veranschaulicht diese Situation anhand der schematischen Wiedergabe des distalen Endes einer Trachealkanüle 1, aus welcher die konische Spitze 8 einer Einführhilfe hervorragt, wobei jedoch der stirnseitige Rand 9 der Trachealkanüle 1 freiliegt und insbesondere an Engstellen des Tracheostomas die Einführung erheblich erschwert.

Gerade bei Kanülen mit dicken Wänden kann dieser abrupte Übergang ein echtes Problem darstellen. So bleibt der Arzt beim Versuch, die Kanüle einzusetzen, häufig mit der Kanülenwand an einer Trachealspange hängen. Dabei kann es zu einer Fraktur des Ringknorpels kommen.

Zwar könnte man auch den Rand 9 der Trachealkanüle 1 in Verlängerung der konischen Spitze 8 abschrägen, jedoch bedeutet dies, daß nach dem Entfernen der Einführhilfe und damit auch der konischen Spitze 8 ein relativ scharfkantiger Innenrand das distale Ende der Trachealkanüle 1 bildet, der wiederum bei Kontakt mit der empfindlichen Trachea Verletzungen oder Wundstellen hervorrufen kann und dadurch auch Schmerzen verursacht, die das dauerhafte Tragen einer solchen Trachealkanüle für den Patienten zusätzlich unangenehm machen.

US 4,502,482 offenbart ein Trachealtubus-Set zum Einführen in die Luftröhre, mit einer stumpfen, atraumatisch geformten Spitze. Eine entfernbare Einführhilfe mit einem konischen Endabschnitt ist weiter vorgesehen, die das Einführen des Trachealtubus durch den Kehlkopf erleichtern soll.

US 5,824,002 betrifft einen Trokar, umfassend ein Schneidelement, eine dieses umgebende isolierende Hülle und eine die Hülle umgebende Kanüle. Die Kanüle weist einen größeren Innendurchmesser auf als der Außendurchmesser der Hülle und ein Übergangselement zwischen Kanüle und Hülle soll beim Einführen den Widerstand an der Vorrichtung verringern. Gegenüber diesem Stand der Technik liegt der vorliegenden Erfindung die Aufgabe zugrunde, eine Vorrichtung zum Einbringen einer Trachealkanüle mit den eingangs genannten Merkmalen zu schaffen, die das Verletzungsrisiko beim Einbringen der Trachealkanüle erheblich reduziert und den Einbringvorgang erleichtert, wobei die so eingebrachte Kanüle auch bei dauerhaftem Gebrauch einen hohen Tragekomfort aufweist und die Gefahr von Verletzungen oder Wundstellen in der Trachea gering hält.

Die Erfindung wird durch eine Vorrichtung mit den Merkmalen des Anspruchs 1 definiert.

Bei dieser Vorrichtungbesteht die konische Spitze aus einem inneren Kern und einer äußeren Hülse, wobei Hülse und Kern an oder in der Nähe des vorderen Endes der konischen Spitze miteinander verbunden sind und wobei die äußere Hülse an der Basis der konischen Spitze eine radiale Dicke hat, die in etwa der Wandstärke der Trachealkanüle an deren vorderem Ende entspricht, und wobei der Kern im Bereich der Basis einen maximalen Durchmesser hat, der kleiner ist als oder gleich ist wie der Innendurchmesser der Trachealkanüle, und wobei der Kern mit einem flexiblen Schaft bzw. Zugelement verbunden ist, das sich durch die einzuführende Trachealkanüle erstreckt, wobei das Ausüben eines Zugs auf das Zugelement entgegen der durch die konische Spitze vorgegebenen Richtung bewirkt, dass die sich am vorderen Ende der Trachealkanüle abstützende Hülse von ihrem Ansatz an dem Kern her umgestülpt wird und sich aufgrund ihrer elastischen Vorspannung zusammenzieht, bis ihr Außendurchmesser in etwa gleich dem oder kleiner als der Innendurchmesser der Trachealkanüle ist, so dass die konische Spitze mit der mindestens teilweise umgestülpten Hülse durch die Trachealkanüle zurückziehbar ist, und wobei die Einführhilfe eine zentrale Bohrung zur Aufnahme eines Führungsdrahtes aufweist.

Dabei soll sowohl der Zustand mit kleinem Basisdurchmesser als auch der Zustand mit großem Basisdurchmesser wahlweise einstellbar sein.

Der Begriff "konisch" ist in diesem Fall nicht im streng geometrischen Sinne zu verstehen, sondern bezieht sich im wesentlichen auf einen von der Spitze zur Basis hin zunehmenden Durchmesser ohne sprunghafte Aufweitungen, wobei die Kontur des "Konus" durchaus konkav oder konvex gewölbt sein kann.

Der Begriff "Basis" der konischen Spitze bezieht sich im Übrigen wörtlich zunächst nur auf die untere Ebene der konischen Spitze, die den maximalen Konusdurchmesser hat, und die das Ausmaß der Abdeckung der distalen Stirnfläche der Kanüle bestimmt. Entsprechend dem jeweiligen Sinnzusammenhang umfasst aber der Begriff "Basis" gegebenenfalls auch den an diese Ebene angrenzenden unteren Abschnitt des Konus, soweit nämlich vor dem Hindurchführen des Konus durch die Kanüle dessen Durchmesser in dem Zustand mit großem Basisdurchmesser ebenfalls noch größer ist als der Innendurchmesser der Kanüle. Zum Zurückziehen der Einführhilfe durch die Kanüle muß auch dieser Bereich (im Zustand der Basis mit kleinem Durchmesser) einen Durchmesser einnehmen, der maximal dem Inndurchmesser der Kanüle entspricht.
Eine solche Einführhilfe wird zunächst in dem Zustand der konischen Spitze mit kleinem Basisdurchmesser von der proximalen Seite her in die Trachealkanüle eingebracht, wobei die konische Spitze spätestens dann in den Zustand mit großem Basisdurchmesser gebracht wird, wenn sie durch die Kanüle hindurch zum distalen Ende geführt worden ist, so daß die konische Spitze vollständig aus dem distalen Ende herausgetreten ist. Alternativ kann eine entsprechend ausgestaltete Einführhilfe vor dem Einbringen in das Tracheostoma auch vom distalen Ende der Trachealkanüle her in diese eingeführt werden Bei der Einführung von dem distalen Ende her kann die konische Spitze von vornherein in dem Zustand mit großem Basisdurchmesser sein. Allerdings muß dann das proximale Ende mit dem Schaft der Einführhilfe durch die Kanüle hindurch passen.

In der Regel sind am proximalen Ende der Einführhilfe Betätigungseinrichtungen vorgesehen, die zumindest teilweise mit der Einführhilfe einstückig verbunden sind und die im allgemeinen zu groß sind, als daß sie durch eine Trachealkanüle hindurchpassen, so daß in der Regel die Einführhilfe von der proximalen Seite her in die Trachealkanüle eingeführt wird. In der Praxis kann eine entsprechende Kanüle mit Einführhilfe vormontiert in steriler Packung geliefert werden.

In dem Zustand mit großem Basisdurchmesser der konischen Spitze wird dann die Trachealkanüle zusammen mit der Einführhilfe in das Tracheostoma eingeführt, wobei die konische Spitze für eine schonende Aufweitung sorgt und das Tracheostoma bis auf den vollen Außendurchmesser der Trachealkanüle erweitert, so daß diese entsprechend leicht eingeführt werden kann, nicht an Engstellen hängenbleibt und auch keinerlei zusätzliche Verletzungen verursacht.

Nachdem die Trachealkanüle eingeführt worden ist und ihre gewünschte Endposition erreicht hat, wird die konische Spitze in den Zustand mit kleinem Basisdurchmesser gebracht, so daß sie in diesem Zustand durch die Trachealkanüle zurückgezogen werden kann, während die Trachealkanüle festgehalten wird und an ihrem Platz verbleibt. Selbstverständlich könnte die konische Spitze auch schon früher in den Zustand mit kleinem Basisdurchmesser gebracht werden, wenn entsprechende Engstellen und insbesondere die Knorpelspangen des Kehlkopfes durchstoßen sind und das distale Ende der Kanüle die Trachea erreicht hat. Die weitere Einführung der Kanüle in die Trachea in ihre endgültige Position kann dann auch ohne die konische Spitze erfolgen, die dementsprechend schon früher in den Zustand mit kleinem Basisdurchmesser gebracht und zurückgezogen werden könnte.

In einer Ausführungsform einer Vorrichtung zum Einbringen einer Tracheostomiekanüle in ein Tracheostoma ist mindestens die konische Spitze, genauer gesagt mindestens die Basis der konischen Spitze und der daran angrenzende Schaftabschnitt der Einführhilfe aus einem elastischen Material hergestellt, welches bei Aufbringen entsprechender Kräfte in den Zustand mit großem Basisdurchmesser gedehnt werden kann und sich bei nachlassenden Kräften aufgrund der inhärenten elastischen Rückstellkräfte wieder in den Zustand mit kleinem Basisdurchmesser zurückzieht. Bei einer solchen Ausführungsform sind die konische Spitze und der angrenzende Schaftabschnitt mit einer im wesentlichen zentralen Längsbohrung versehen, wobei ein Verdrängungskörper vorgesehen ist, welcher einen deutlich größeren Durchmesser hat als die zentrale Längsbohrung (im unbelasteten Zustand) und welcher in dieser Bohrung bis in den Bereich der konischen Spitze hinein verschiebbar und auch wieder zurückziehbar ist, so daß beim Hineinschieben des Verdrängungskörpers in die Bohrung die konische Spitze in den Zustand mit großem Basisdurchmesser aufgeweitet wird und nach dem Herausziehen des Verdrängungskörpers aus diesem Bereich der Bohrung sich die konische Spitze wieder in den Zustand mit kleinerem Basisdurchmesser zusammenzieht.

Eine ähnliche Ausführungsform weist eine in mehrere Sektoren aufgeteilte konische Spitze auf, die von einer äußeren, elastischen Hülle umfaßt und zusammengehalten werden und vorzugsweise zusätzlich auch an der Spitze des Konus zusammenhängen. Die einzelnen Sektorelemente umschließen ihrerseits einen Hohlraum eine, der wie zuvor durch einen entsprechenden Verdrängungskörper aufgeweitet werden kann, wobei die einzelnen Sektoren nach außen gedrängt werden und dabei die äußere elastische Hülle dehnen, bis die konische Spitze insgesamt den Zustand mit großem Durchmesser erreicht hat. Nach dem Zurückziehen des Verdrängungskörpers sorgt die elastische Hülle dafür, daß die einzelnen Sektorelemente wieder zusammengedrückt werden, so daß die konische Spitze insgesamt einen Zustand mit kleinem Durchmesser erreicht.

Bei einer solchen Ausführungsform braucht der an die konische Spitze anschließende Abschnitt des Schaftes der Einführhilfe als solcher nicht elastisch aufweitbar zu sein, sondern kann eine konstanten, unveränderlichen Durchmesser aufweisen, der maximal dem Innendurchmesser der Kanüle entspricht.

Bei der Vorrichtung zum Einbringen einer Trachealkanüle in ein Tracheostoma besteht die konische Spitze aus einem inneren Kern und einer äußeren Hülse aus einem flexiblen Material, wobei Hülse und Kern am vorderen Ende der konischen Spitze zusammenhängen und wobei die äußere Hülse an der Basis der konischen Spitze eine radiale Dicke hat, welche mindestens in etwa der halben Wandstärke der zugehörigen Trachealkanüle entspricht oder auch gegebenenfalls etwas größer ist, während der Kern einen maximalen Durchmesser hat, der höchstens gleich dem Innendurchmesser der Trachealkanüle ist. Dieser Kern ist mit einem flexiblen Schaft verbunden, durch welchen der Kern der Trachealkanüle in das Innere der Kanüle zurückziehbar ist. Da die Spitze des Kerns mit der äußeren, elastischen Hülle zusammenhängt, wird durch Zurückziehen des Kerns die konische Spitze nach innen eingestülpt, während die Basis der äußeren elastischen Hülse sich nach wie vor an dem vorderen Rand der Trachealkanüle abstützt. Die Innenfläche der äußeren elastischen Hülse liegt dabei an der Außenfläche des Kerns an und die Hülse kann sich immer weiter zusammenziehen, wenn der Kern zurückgezogen wird, da die Basis der äußeren Hülse an Kernbereichen mit zunehmend kleinerem Durchmesser anliegt, während der Kern zurückgezogen wird. Schließlich ist ein Zustand erreicht, in welchem der Kern so weit zurückgezogen ist, daß der Außendurchmesser der an dem Kern der konischen Spitze anliegenden Basis der äußeren Hülse kleiner ist als der Innendurchmesser der Trachealkanüle. In diesem Zustand gleitet bei weiterem Zurückziehen des Kerns auch die Hülse mit in die Trachealkanüle hinein und die Einführhilfe kann dann vollständig aus der Kanüle zurückgezogen werden. Die Funktionsweise beim Einbringen der Trachealkanüle ist praktisch die gleiche wie im Falle der zunächst beschriebenen Ausführungsform. Lediglich die Art und Weise, wie die konische Spitze aus dem Zustand mit großem Basisdurchmesser in den Zustand mit kleinem Durchmesser gebracht wird, unterscheidet sich von der zuvor beschriebenen Ausführungsform.

Ein Vorteil der vorstehend beschriebenen Variante besteht darin, daß die konische Spitze sich beim Zurückziehen des Schaftes und des Kernes auf keinen Fall an der distalen Stirnseite der Kanüle verhaken kann, da die äußere Hülse, selbst wenn sie zunächst mit Ihrer Basis an der Stirnseite der Kanüle anliegt und sich nicht hinreichend zusammenzieht, um in dieser Ausrichtung in die distale Öffnung die Kanüle hinein zu rutschen, letztlich vollständig umgestülpt wird, so daß die Basisfläche dann in die entgegengesetzte Richtung weist und die konische Spitze der Hülse in Richtung der distalen Kanülenöffnung zeigt. In diesem Zustand kann die Einführhilfe mit der umgestülpten Hülse auf jeden Fall in das distale Ende der Kanüle hineingezogen werden, wobei die Hülse notfalls elastisch zusammengedrückt wird.

Bei der zuletzt genannten Ausführungsform kann selbstverständlich der Kern aus einem vergleichsweise starren und steifen Material gefertigt sein, während die Hülse zumindest im Bereich ihrer Basis und den angrenzenden Abschnitten hinreichend elastisch sein muß, damit sie sich, wenn der Kern den Innendurchmesser entsprechend freigibt, insgesamt auf einen Durchmesser zusammenziehen oder in einen Zustand zusammengedrückt werden kann, der höchstens dem Innendurchmesser der Kanüle entspricht.

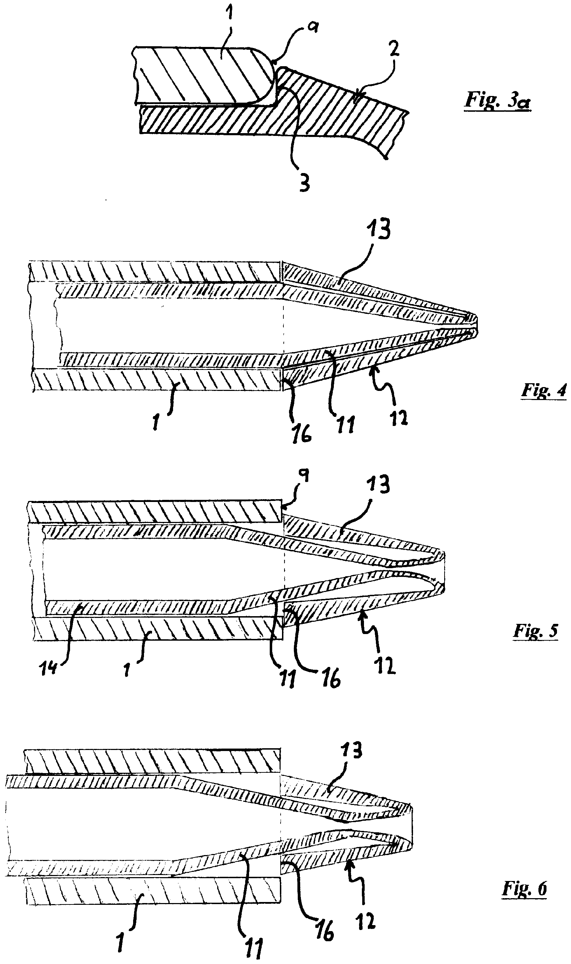

Bei den Ausführungsformen der Erfindung weist die Einführhilfe (einschließlich etwaiger Betätigungselemente, soweit sie zentral angeordnet sind, wie zum Beispiel der Verdrängungskörper nach den Figuren 2 und 3) jeweils eine zentrale Bohrung zur Aufnahme eines Führungsdrahtes auf. Die erfindungsgemäße Einführhilfe ist dann sogar geeignet, herkömmliche Dilatatoren vollständig zu ersetzen, weil die konische Spitze der Einführhilfe bereits die Funktion eines Dilatators ausübt.

Weitere Vorteile, Merkmale und Anwendungsmöglichkeiten der vorliegenden Erfindung werden deutlich anhand der folgenden Beschreibung einer bevorzugten Ausführungsform und der dazugehörigen Figuren. Es zeigen:
- Figur 1: eine Einführhilfe mit einer Trachealkanüle nach dem Stand der Technik,
- Figur 2: eine Ausführungsform einer Einführhilfe mit einer Trachealkanüle in einem Zustand mit großem Basisdurchmesser der konischen Spitze,
- Figur 3: die Ausführungsform nach Figur 2 mit einem kleinen Durchmesser der konischen Spitze,
- Figur 3a: eine Detailansicht der Basis einer konischen Spitze der Einführhilfe und des distalen Endes einer Trachealkanüle einer weiteren Ausführungsform ähnlich der in Figur 2 dargestellten Ausführungsform,
- Figur 4: eine erfindungsgemäße Ausführungsform mit einem inneren Kern und einer äußeren elastischen Hülse, die durch Zurückziehen des Kerns einstülpbar ist,
- Figur 5: die Ausführungsform nach Figur 4 mit einem teilweise zurückgezogenen Kern und
- Figur 6: die Ausführungsform nach Figur 4 bei einem hinreichend zurückgezogenen Kern, so daß die teilweise umgestülpte äußere Hülse an der Basis einen Außendurchmesser hat, der dem Innendurchmesser der Kanüle entspricht,
- Figur 7: eine Variante zu der in den Figuren 4 - 6 dargestellten Ausführungsform und
- Figur 8: die Variante gemäß Figur 7 in einem zurückziehbaren Zustand.

In Figur 1 erkennt man die mit 1 bezeichnete Trachealkanüle in einem Längsschnitt, wobei sich eine Einführhilfe in Form eines flexiblen Kunststoffstabs oder vorzugsweise eines Kunststoffschlauchs mit einer konischen Spitze 8 durch das Innere der Trachealkanüle erstreckt und am distalen Ende aus der Trachealkanüle 1 herausragt. Weil nach dem Einbringen der Kanüle 1 die konische Spitze 8 zurückgezogen werden muß und keine Möglichkeit zur Verringerung dieses Durchmessers vorgesehen ist, entspricht der Durchmesser der konischen Spitze an der Basis in etwa dem Innendurchmesser der Trachealkanüle 1, so daß die Stirnseite 9 des distalen Endes der Trachealkanüle, die durch das Tracheostoma eingeführt werden muß, freiliegt.

Es versteht sich, daß alle Figuren die einzelnen Elemente und Merkmale nur schematisch wiedergeben und daß zum Beispiel das Verhältnis der Wandstärke zum Innendurchmesser der Trachealkanüle 1 in den hier vorliegenden Figuren übertrieben dargestellt ist. Auch müssen die Kanten an den Stirnseiten 9 der Trachealkanüle 1 nicht wirklich scharfkantig mit im Querschnitt rechtwinkligen Ecken ausgebildet sein, sondern können mit einem kleinen Radius gerundet sein. Dennoch ergibt sich bei den bekannten Einführhilfen letztlich immer ein stufenförmiger Übergang von der Basis der konischen Spitze 8 oder des anschließenden Schaftabschnitts der Einführhilfe zu der Stirnseite 9 der Trachealkanüle 1.

Figur 2 zeigt eine Ausführungsform in dem Zustand der konischen Spitze mit großem Basisdurchmesser. Wie man erkennt, besteht die Einführhilfe aus einem rohr- oder schlauchförmigen Schaft 6, dessen zentrales Lumen bzw. zentrale Bohrung 5 sich bis weit in die konische Spitze 2 der Einführhilfe erstreckt und in der konischen Spitze 2 als Sackbohrung bzw. Sackloch endet. Figur 3 zeigt dieselbe Ausführungsform in einem Zustand der konischen Spitze 2 mit kleinem Basisdurchmesser.

Wie man sieht, ist in der zentralen Bohrung 5 ein Verdrängungskörper 4 axial beweglich angeordnet. In dem in Figur 3 dargestellten Zustand ist dieser Verdrängungskörper 4 ein Stück weit in den Schaft 6 der Einführhilfe zurückgezogen, und zwar bis in einen Bereich des Schaftes 6, der zwar ausreichend flexibel ist, um sich der Form der Trachealkanüle anzupassen, der aber keine besondere Elastizität aufweisen muß und sich deshalb auch nicht oder nicht nennenswert zusammenzieht, wenn der Verdrängungskörper 4 in diesen Bereich oder aus diesem Bereich zurückgezogen wird. Das vordere Ende 6' des Schaftes jedoch, welches an die Basis 3 der konischen Spitze 2 anschließt, ist aus einem hinreichend elastischen Material gefertigt und nimmt in einem von äußeren Kräften freien Zustand die in Figur 3 dargestellte Form an, in welcher auch der Durchmesser der Basis 3 der konischen Spitze 2 höchstens gleich dem Innendurchmesser der Trachealkanüle ist. Mit "äußere Kräfte" sind hier nicht geometrisch von der radial äußeren Seite der Einführhilfe wirkende Kräfte gemeint, sondern alle Kräfte, die nicht wie die elastischen Kräfte des Materials der Einführhilfe inhärente Kräfte derselben sind, sondern vielmehr alle Kräfte, die zwangsweise von außerhalb des Materials der Einführhilfe auf diese aufgebracht werden. Insbesondere sind also auch die durch den Verdrängungskörper 4 auf die Innenwände der Bohrung 5 aufgebrachten Kräfte "äußere Kräfte" in dem vorstehend erläuterten Sinne. Der Zustand nach Figur 3 ist demnach der (von äußeren Kräften freie oder kurz:) "kräftefreie" Zustand.

In der Praxis wird der Durchmesser der Basis 3 im kräftefreien Zustand etwas kleiner gewählt als der Innendurchmesser der Trachealkanüle 1. Wenn die Trachealkanüle 1 in ein Tracheostoma eingebracht werden soll, muß die konische Spitze 2 sich in dem in Figur 2 dargestellten Zustand befinden. Zu diesem Zweck wird der Verdrängungskörper 4 in der Bohrung 5 axial nach vorn bis in die konische Spitze 2 hineinbewegt. Dabei werden der flexible Schaftabschnitt 6' ebenso wie die gesamte Basis und der angrenzende Abschnitt der konischen Spitze aufgeweitet, weil der Durchmesser des Verdrängungskörpers 4 wesentlich größer ist als der Durchmesser der Bohrung 5 im Bereich der konischen Spitze 2 in dem kräftefreien Zustand, wie er in Figur 3 dargestellt ist. Demzufolge weitet sich der Durchmesser der Basis 3 auf, wobei der Verdrängungskörper 4 und die Bohrung 5 so bemessen sind, daß der Basisdurchmesser in dem in Figur 2 dargestellten Zustand im wesentlichen dem Außendurchmesser der Trachealkanüle entspricht.

Unabhängig davon könnten die Außenkante des distalen Endes oder die gesamte distale Stirnseite 9 der Trachealkanüle 1 abgerundet sein ebenso wie auch die äußere Kante der Basis der konischen Spitze etwas abgerundet sein kann, um das Hineinziehen der konischen Spitze in die distale Öffnung der Trachealkanüle und das Zurückziehen durch das Lumen der Trachealkanüle zu erleichtern. In diesem Fall braucht der Durchmesser der Basis der konischen Spitze beispielweise nur etwa ein Maß entsprechend dem Mittelwert zwischen Innen- und Außendurchmesser des distalen Endes der Kanüle oder etwas mehr zu haben, wie dies beispielhaft in einer Detailansicht gemäß Figur 3a zu erkennen ist. In diesem Beispiel folgt der äußere Teil der Abrundung der Stirnseite 9 der Kanüle 1 in etwa der Verlängerung des konischen Verlaufs der Spitze 2.

Des Weiteren ist in den Figuren 2 und 3 mit gestrichelten Linien eine zentrale Bohrung 10 angedeutet, die in einer bevorzugten Variante dieser Ausführungsform vorgesehen ist. Diese Bohrung 10, die in ähnlicher Weise auch bei den noch zu beschreibenden Ausführungsformen inhärent vorhanden ist, dient zur Aufnahme eines Führungsdrahtes, auf welchen die Einführhilfe aufgeschoben werden kann.

Eine Ausführungsform einer erfindungsgemäßen Trachealkanüle ist in den Figuren 4 bis 6 dargestellt. In diesem Fall besteht die konische Spitze aus einem inneren Kern 11 aus einem relativ festen und steifen Material und einer äußeren Hülse 13, die aus einem sehr flexiblen, elastischen Material hergestellt ist. Der Kern 11 und die äußere Hülse 13 hängen nur am vorderen Ende der konischen Spitze zusammen, wobei die Spitze in diesem Zustand eine etwas abgerundete Form mit einer leichten zentralen Vertiefung hat, was aber deren Gebrauch nicht beeinträchtigt, wenn diese Spitze insgesamt einen hinreichend kleinen Durchmesser hat.

In dem in Figur 4 dargestellten Zustand wird die Trachealkanüle 1 in genau der gleichen Weise in das Tracheostoma eingebracht, wie dies im Zusammenhang mit der Einführhilfe nach Figur 2 beschrieben wurde. Die konische Spitze weitet etwaige Engstellen, insbesondere im Bereich der Knorpelspangen des Kehlkopfes, aufgrund des sanften konischen Übergangs auf, bis diese Engstellen auf den Außendurchmessern der Trachealkanüle 1 aufgeweitet sind, so daß die Trachealkanüle 1 entsprechend nachgeschoben werden kann. Sobald das distale Ende der Trachealkanüle 1 das Innere der Trachea bzw. seine endgültige Position erreicht hat, kann die Einführhilfe zurückgezogen werden, indem der innere Kern 11 mit Hilfe des angrenzenden Schafts 14, der als Schlauch oder flexibles Rohr ausgebildet ist, zurückgezogen wird. Die Basis 16 der äußeren Hülse 13 stützt sich dabei an der Stirnseite 9 der Trachealkanüle 1 ab, so daß sich die äußere Hülse 13 im Bereich der konischen Spitze notwendigerweise einstülpen bzw. umstülpen muß. Da die äußere Hülse 13 aus einem elastischen Material hergestellt ist, zieht sich währenddessen die Basis 16 in radialer Richtung zusammen, da sie auf Ihrer Innenseite nach dem Zurückziehen des konischen Kerns 11 durch Kernbereiche abgestützt wird, die einen immer kleiner werdenden Durchmesser haben. In Figur 5 ist ein Zwischenzustand des Umstülpens der Hülse 13 und Zurückziehens des Kerns 11 dargestellt. In dem in Figur 6 dargestellten Zustand ist der Kernbereich 11 so weit zurückgezogen, daß die sich elastisch zusammenziehende äußere Hülse 13, die mit ihrer Basis 16 an einem Bereich des Kerns 11 anliegt, der einen entsprechend geringen Durchmesser hat, so daß der Außendurchmesser der Basis 16 der Hülse 13 nunmehr den Wert des Innendurchmessers der Trachealkanüle 1 erreicht hat. In diesem Zustand kann die Einführhilfe 10 durch die Trachealkanüle 1 hindurch und am proximalen Ende herausgezogen werden.

Sofern die Hülse 13 sich während des eben beschriebenen Vorgangs nicht hinreichend zusammenzieht, oder die Hülse sich exzentrisch verzieht oder verkippt, so daß die Basis 16 der Hülse 13 weiterhin mit der Stirnseite 9 der Kanüle 1 in Kontakt bleibt, führt dies beim weiteren Zurückziehen des Schafts 14 und des Kerns 11 letztlich dazu, daß die Hülse 13 vollständig umgestülpt wird. In diesem Fall weist die Basis 16 der Hülse gegenüber der Darstellung in den Figuren 4 bis 6 in die entgegengesetzte Richtung und die Spitze der konischen Hülse 13 weist ebenfalls in die gegenüber Figur 4 entgegengesetzte Richtung. In diesem Zustand läßt sich die Hülse 13 auch vollständig in die distale Öffnung der Kanüle 1 hineinziehen, selbst wenn der Außendurchmesser der Basis 16 der elastischen Hülse 13 noch immer größer sein sollte, als es dem Innendurchmesser der Kanüle 1 entspricht. In diesem Fall würde aufgrund der konischen Außenfläche der Hülse 13, die vormals die Innenfläche dieser Hülse war und die mit dem Rand der distalen Öffnung mit der Kanüle 1 in Kontakt tritt, die Hülse 13 hinreichend zusammengedrückt werden, um in die Kanüle 1 hinein- und am proximalen Ende herausgezogen werden zu können.

Geeignete Handhabungseinrichtungen für die Einführhilfe bzw. das proximale Ende des Schaftes 6 bzw. 14 ergeben sich aus der beschriebenen Funktionsweise für das Einstellen der verschiedenen Zustände der konischen Spitze in naheliegender Weise und brauchen hier nicht weiter beschrieben zu werden. Beispielsweise könnte das proximale Ende des Schafts 6 bzw. 14 mit einem ringförmigen Greifabschnitt versehen werden, in welchem ein Finger eingeführt werden kann, um die Einführhilfe zurückzuziehen, während die Trachealkanüle 1 an ihrem Platz gehalten wird. Auch für den Verdrängungskörper 4 gemäß den Ausführungsformen der Figuren 2 und 3 kann eine entsprechende Betätigung in Form eines Greifrings oder dergleichen vorgesehen sein. Statt eines Greifrings könnten aber auch Flansche oder andere Einrichtungen vorgesehen sein, die das Ergreifen der Einführhilfen bzw. des Verdrängungskörpers erleichtern.

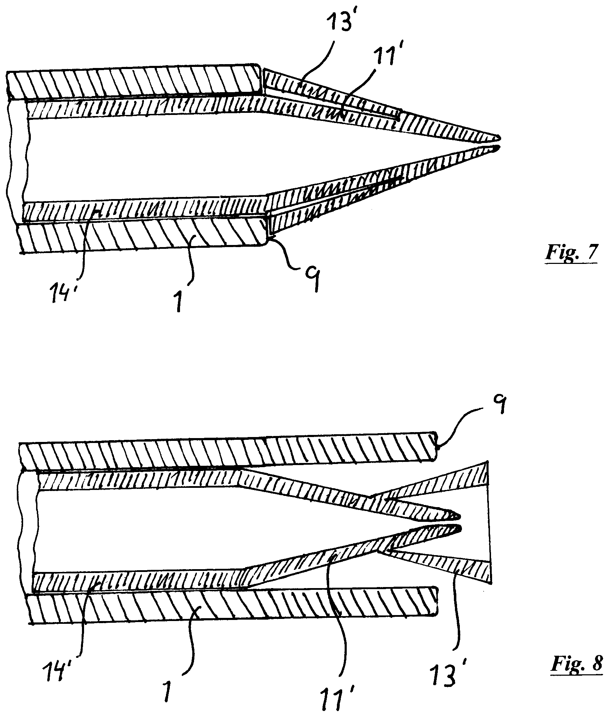

Eine Variante zu der in den Figuren 4 bis 6 dargestellten Ausführungsform zeigen die Figuren 7 und 8. In diesem Fall setzt die äußere Hülse 13' nicht an der Spitze des konischen Kerns 11' an, sondern im Abstand zu dieser Spitze. Die Funktionsweise dieser Ausführungsform ist jedoch dieselbe wie sie im Zusammenhang mit den Figuren 4 bis 6 geschildert wurde. Auch hier ist die Darstellung nur schematisch und etwaige zeichnerische Ungenauigkeiten hinsichtlich der Maße und genauen Positionierung einzelner Elemente in den Figuren 7 und 8 (ebenso wie auch in den vorher beschriebenen Figuren) sind nicht im Gegensatz zu den Aussagen der Beschreibung und der Ansprüche zu interpretieren. So liegt beispielsweise die Basis der äußeren Hülse 13' in dem in Figur 7 dargestellten Zustand aufgrund ihrer Elastizität eng an dem inneren konischen Kern 11' an, auch wenn zur Verdeutlichung der beiden Elemente als gegeneinander bewegliche Teile in der Zeichnung ein Abstand zwischen der Basis der Hülse und dem Kern vorhanden zu sein scheint. Da die Hülse 13' im Vergleich zu der Hülse 13 der Figuren 4 bis 6 axial kürzer ausgebildet ist und an einem Abschnitt des Kerns 11' mit größerem Durchmesser ansetzt, hat sie beim Zurückziehen des Schaftes 14 in die Kanüle 1 gegenüber der Ausführungsform nach den Figuren 4 bis 6 eher die Tendenz, sich vollständig umzustülpen bzw. umzuklappen, wie dies in Figur 8 dargestellt ist.

## Patentansprüche

1. Vorrichtung zum Einbringen einer Trachealkanüle in ein Tracheostoma, mit einer Einführhilfe, die einen durch die Trachealkanüle hindurchführbaren Schaft (6, 6', 14) und eine mit dem Schaft verbundene konische Spitze (2, 13) aufweist, die in einen ersten Zustand mit einem kleinen Basisdurchmesser und einen zweiten Zustand mit großem Basisdurchmesser bringbar ist, wobei die Einführhilfe einen an die konische Spitze (2) angrenzenden Abschnitt (6', 14) hat, dessen Durchmesser maximal dem Innendurchmesser einer mit der Einführhilfe einzuführenden Trachealkanüle (1) entspricht und wobei der kleine Basisdurchmesser der konischen Spitze ebenfalls höchstens gleich dem Innendurchmesser der Trachealkanüle ist, während der große Basisdurchmesser der konischen Spitze größer als der Innendurchmesser der Trachealkanüle ist und vorzugsweise in etwa dem Außendurchmesser der damit einzubringenden Trachealkanüle entspricht, wobei die konische Spitze aus einem inneren Kern (11, 11') und einer äußeren Hülse (13, 13') besteht, wobei Hülse (13) und Kern (11) am oder in der Nähe des vorderen Endes der konischen Spitze miteinander verbunden sind und wobei die äußere Hülse an der Basis der konischen Spitze eine radiale Dicke hat, die in etwa der Wandstärke der Trachealkanüle an deren vorderem Ende entspricht, und wobei der Kern (11, 11') im Bereich der Basis einen maximalen Durchmesser hat, der kleiner ist als oder gleich ist wie der Innendurchmesser der Trachealkanüle (1), und wobei der Kern (11, 11') mit einem flexiblen Schaft bzw. Zugelement (14, 14') verbunden ist, das durch die Trachealkanüle hindurchführbar ist, wobei das Ausüben eines Zugs auf das Zugelement entgegen der durch die konische Spitze vorgegebenen Richtung bewirkt, dass die sich am vorderen Ende der Trachealkanüle abstützende Hülse von ihrem Ansatz an dem Kern her umgestülpt wird und sich aufgrund ihrer elastischen Vorspannung zusammenzieht, bis ihr Außendurchmesser in etwa gleich dem oder kleiner als der Innendurchmesser der Trachealkanüle ist, so dass die konische Spitze mit der mindestens teilweise umgestülpten Hülse (13) durch die Trachealkanüle zurückziehbar ist, und wobei die Einführhilfe eine zentrale Bohrung zur Aufnahme eines Führungsdrahtes aufweist.

2. Vorrichtung nach Anspruch 1, **dadurch gekennzeichnet, dass** der innere Kern (11') konisch ist und dass die Hülse (13') und der konische Kern (11') in einem Abstand von der konischen Spitze miteinander verbunden sind, der in etwa ¼ bis ½ der axialen Länge des konischen Kernes entspricht.

3. Vorrichtung nach Anspruch1 oder 2, **dadurch gekennzeichnet, dass** der innere Kern (11') und die äußere Hülse (13') so ausgestaltet und miteinander verbunden sind, dass das Ausüben eines Zugs auf das Zugelement (14') entgegen der durch die konische Spitze vorgegebenen Richtung bewirkt, dass die sich am vorderen Ende der Trachealkanüle (1) abstützende Hülse (13') sich zusammenzieht und von ihrem Ansatz an dem Kern her vollständig umgestülpt wird, so dass die konische Spitze mit der umgestülpten Hülse (13') durch die Trachealkanüle zurückziehbar ist.

## Claims

1. Device for inserting a tracheostomy tube into a tracheostoma, with an insertion aid which has a stem (6, 6', 14) that can be passed through the tracheostomy tube and a conical tip (2, 13) that is connected to the stem, which can be changed into a first state with a small base diameter and a second state with a large base diameter, wherein the insertion aid has a section (6', 14) adjacent to the conical tip (2), the diameter of which corresponds at most to the internal diameter of a tracheostomy tube (1) to be inserted with the insertion aid and wherein the small base diameter of the conical tip is likewise at most equal to the internal diameter of the tracheostomy tube whereas the large base diameter of the conical tip is greater than the internal diameter of the tracheostomy tube and preferably corresponds approximately to the external diameter of the tracheostomy tube to be inserted with it, wherein the conical tip consists of an inner core (11, 11') and an outer sleeve (13, 13'), wherein sleeve (13) and core (11) are connected to each other on or in the vicinity of the front end of the conical tip and wherein the outer sleeve on the base of the conical tip has a radial thickness which corresponds approximately to the wall thickness of the tracheostomy tube at its front end, and wherein the core (11, 11') has in the area of the base a maximum diameter which is smaller than or equal to the internal diameter of the tracheostomy tube (1), and wherein the core (11, 11') is connected to a flexible stem or traction element (14, 14') which can be passed through the tracheostomy tube to be inserted, wherein the effect of the exertion of traction on the traction element against the direction predetermined by the conical tip is that the sleeve supported on the front end of the tracheostomy tube is inverted from its point of attachment to the core and contracts due to its elastic pre-tension until its external diameter is approximately equal to or smaller than the internal diameter of the tracheostomy tube, with the result that the conical tip with the at least partly inverted sleeve (13) can be withdrawn through the tracheostomy tube, and wherein the insertion aid has a central bore for receiving a guide wire.

2. Device according to claim 1, **characterized in that** the inner core (11') is conical and that the sleeve (13') and core (11') are connected to each other at a distance from the conical tip which corresponds to approximately ¼ to ½ of the axial length of the conical core.

3. Device according to claim 1 or 2, **characterized in that** the inner core (11') and the outer sleeve (13') are designed and connected to each other such that the effect of a pull on the traction element (14') against the direction predetermined by the conical tip is that the sleeve (13') supported on the front end of the tracheostomy tube (1) contracts and is completely inverted from the point of attachment to the core, with the result that the conical tip with the inverted sleeve (13') can be withdrawn through the tracheostomy tube.

## Revendications

1. Dispositif pour l'introduction d'une canule trachéale dans un trachéostome, avec un auxiliaire d'introduction qui comprend une tige (6, 6', 14) conçue pour pouvoir être passée par la canule trachéale et une pointe conique (2, 13) attachée à la tige qui est conçue pour être mise dans un premier état avec un petit diamètre de base et dans un deuxième état avec un grand diamètre de base, l'auxiliaire d'introduction comprenant une section (6', 14) attenante à la pointe conique (2) dont le diamètre correspond au maximum au diamètre intérieur d'une canule trachéale (1) destinée à être introduite à l'aide de l'auxiliaire d'introduction et où le petit diamètre de base de la pointe conique est, lui aussi, au maximum égal au diamètre intérieur de la canule trachéale, alors que le grand diamètre de base de la pointe conique est plus grand que le diamètre intérieur de la canule trachéale et correspond de préférence approximativement au diamètre extérieur de la canule trachéale destinée à être introduite à l'aide de celui-ci, la point conique consistant en un noyau intérieur (11, 11') et une douille extérieure (13, 13'), la douille (13) et le noyau (11) étant attachés l'un à l'autre à, ou proche de, l'extrémité avant de la pointe conique et la douille extérieure présentant à la base de la pointe conique une épaisseur radiale qui correspond approximativement à l'épaisseur de la paroi de la canule trachéale à l'extrémité avant de celle-ci, et où le noyau (11, 11') présente dans la zone de la base un diamètre maximal qui est inférieur ou égal au diamètre intérieur de la canule trachéale (1) et où le noyau (11, 11') est attaché à une tige flexible ou un élément de traction (14, 14') qui est conçu pour pouvoir être passée par la canule trachéale, l'exercice, sur l'élément de traction, d'une traction à l'encontre de la direction prédéterminée par la pointe conique ayant pour effet que la douille qui est en appui sur l'extrémité avant de la canule trachéale, est retroussée à partir de son appui sur le noyau et qu'elle se contracte en raison de sa précontrainte élastique jusqu'à ce que son diamètre extérieur soit approximativement égal ou inférieur au diamètre intérieur de la canule trachéale, si bien que la pointe conique, avec la douille (13) au moins partiellement retroussée, puisse être rétractée à travers la canule trachéale et où l'auxiliaire d'introduction comprend une lumière centrale pour recevoir un fil de guidage.

2. Dispositif selon la revendication 1, **caractérisé en ce que** le noyau intérieur (11') est conique et **en ce que** la douille (13') et le noyau conique (11') sont reliés l'un à l'autre à une distance de la pointe conique qui correspond approximativement à ¼ à ½ de la longueur axiale du noyau conique.

3. Dispositif selon la revendication 1 ou 2, **caractérisé en ce que** le noyau intérieur (11') et la douille extérieure (13') sont conformés et attachés l'un à l'autre de façon que l'exercice, sur l'élément de traction (14'), d'une traction à l'encontre de la direction prédéterminée par la pointe conique ait pour effet que la douille (13') qui est en appui sur l'extrémité avant de la canule trachéale (1), se contracte et soit retroussée entièrement à partir de son appui sur le noyau, si bien que la pointe conique, avec la douille (13') retroussée, puisse être rétractée à travers la canule trachéale.
